# EUROPEAN PATENT SPECIFICATION

(11) **EP 0 973 863 B1**
(45) Date of publication and mention of the grant of the patent: **25.05.2005**
(21) Application number: 97935341.4
(22) Date of filing: 08.08.1997
(51) Int. Cl.: C12M 1/20, B01L 3/00

(54) **METHOD AND DEVICES FOR PARTITIONING BIOLOGICAL SAMPLE LIQUIDS INTO MICROVOLUMES**
VERFAHREN UND VORRICHTUNGEN ZUR UNTERTEILUNG BIOLOGISCHER PROBENFLÜSSIGKEITEN IN MIKROFLÜSSIGKEITSMENGEN
PROCEDE ET DISPOSITIFS POUR DIVISER DES LIQUIDES D'ECHANTILLONS BIOLOGIQUES EN MICROVOLUMES

(30) Priority: 09.04.1997 US 838397
(43) Date of publication of application: 26.01.2000
(73) Proprietor: MINNESOTA MINING AND MANUFACTURING COMPANY, St. Paul, Minnesota 55133-3427 (US)
(72) Inventor: WILLIAMS, Michael, G., Saint Paul, MN 55133-3427 (US); HALVERSON, Kurt, J., Saint Paul, MN 55133-3427 (US); KREJCAREK, Gary, E., Saint Paul, MN 55133-3427 (US); WEI, Ai-Ping, Saint Paul, MN 55133-3427 (US); BERG, James, G., Saint Paul, MN 55133-3427 (US); WICKERT, Peter, D., Saint Paul, MN 55133-3427 (US); CALHOUN, Clyde, D., Saint Paul, MN 55133-3427 (US); DEBE, Mark, K., Saint Paul, MN 55133-3427 (US); QIU, Jun, Saint Paul, MN 55133-3427 (US)
(74) Representative: VOSSIUS & PARTNER
(86) International application number: PCT/US1997/014134
(87) International publication number: WO 1998/045406

(56) References cited:
- WO-A-97/12242
- DE-A- 3 631 066
- DE-A- 3 732 142
- US-A- 4 803 154
- US-A- 5 229 163
- US-A- 5 503 803
- PATENT ABSTRACTS OF JAPAN vol. 017, no. 054 (P-1480), 3 February 1993 & JP 04 265860 A (SEKISUI CHEM CO LTD), 22 September 1992,
- PATENT ABSTRACTS OF JAPAN vol. 012, no. 334 (P-756), 8 September 1988 & JP 63 096558 A (NITTO ELECTRIC IND CO LTD), 27 April 1988,

## Description

This invention relates to methods and devices for partitioning biological samples into microvolume aliquots, based on the tendency for aqueous liquids to be retained within hydrophilic zones of the devices while being substantially excluded from hydrophobic areas of the devices.

The detection and enumeration of microorganisms is practiced in numerous settings, including the food-processing industry (testing for the contamination of food by microorganisms such as *E. coli* and *S. aureus*), the health care industry (testing of patient samples and other clinical samples for infection or contamination), environmental testing industry, the pharmaceutical industry, and the cosmetic industry.

Growth-based detection and enumeration of microorganisms is commonly practiced using either liquid nutrient media (most probable number analysis (MPN)) or semi-solid nutrient media (agar petri dishes). Enumeration using the liquid MPN method is typically achieved by placing serial 10-fold dilutions of a sample of interest in replicate sets of tubes containing selective media and chemical indicators. The tubes are incubated at elevated temperature (24-48 hours) followed by examination for growth of organisms. A statistical formula, based on the number of positive and negative tubes for each set, is used to estimate the number of organisms present in the initial sample.

This method of performing MPN analysis has several disadvantages. It is labor intensive because of the multiple diluting and pipetting steps necessary to perform the analysis. In addition, in practice it is only practical to use replicate sets of about three to five tubes for each dilution. As a result, the 95% confidence limits for an MPN estimate for microbial concentration are extremely wide. For example, a three tube MPN estimate of 20 has 95% confidence limits ranging from 7 to 89.

In contrast to the method described above, a direct count of viable microorganisms in a sample can be achieved by spreading the sample over a defined area using nutrient media containing a gelling agent. The gelling agent (agar) prevents diffusion of the organisms during incubation (24-48 hours), producing a colony in the area where the original organism was deposited. There is, however, a limit to the number of colonies that can fit on a given area of nutrient media before fusion with neighboring colonies makes counting difficult. This usually necessitates performing several dilutions for each sample. In addition, the classes of chemical indicator molecules that can be used for identifying individual types of microorganisms present within a mixed population are limited to those that produce a product that is insoluble in the gelled media.

In addition to these disadvantages, both the currently used MPN analysis and gel-based systems require a relatively long incubation time before a positive result can be detected.

DE-A-3732142 is directed to a manufacturing method of a glass element with a mask and a stamp for carrying out the manufacturing method. In particular, hydrophilic areas are created by means of stamp hydrophobic areas.

US-A-5,229,163 is directed to a microtiter plate containing a plurality of reaction wells for conducting immunogenic reactions. The bottom wall of the reaction well has an inner surface which is substantially hydrophilic, and a side wall of the reaction well has an inner surface which is substantially hydrophobic.

GB-A-2 106 539 discloses a bacteriological most probable number strip for the enumeration of microorganisms in liquids or suspensions, wherein a biological sample can be separated into volumes of one 50ml, five 10ml and five 1ml.

The invention is based on the discovery that biological liquid samples can be partitioned into discrete microvolumes with only minimal manipulation on the part of an operator. The method of partitioning employs devices that have hydrophilic liquid-retaining zones surrounded by hydrophobic "land" areas. The methods and devices provide a system for the detection and enumeration of microorganisms and other biological materials that solves the problems associated with currently used systems. The system is a liquid-based system, allowing efficient and effective partitioning of the sample into discrete microvolumes for testing, and allows for rapid detection and enumeration.

In the case of MPN analysis for the detection and enumeration of microorganisms, the approaches described herein allow for the use of water-soluble indicator species, and reduce or eliminate the need for the several dilutions typically required in current MPN analysis.

In general, the invention features a method for partitioning an aqueous liquid sample, into discrete microvolumes, comprising
a) providing a device for culturing a microorganism, said device having an assay surface, the assay surface comprising hydrophilic liquid-retaining zones and a hydrophobic land area between the zones, each zone having a microvolume capacity of liquid retention; and
b) contacting the liquid sample with the assay surface such that the liquid sample is partitioned into the hydrophilic liquid-retaining zones.

The zones may comprise a coating or deposition of assay reagent, such as a nutrient medium or indicator substance. Appropriate indicator substances include without limitation chromogenic indicators, fluorescent indicators, luminescent indicators and electrochemical indicators. For purposes of this application, the term "electrochemical" means a chemical indicator that changes the resistance or conductance of the sample upon reaction with the microorganism.

The zones may be of uniform size, with each zone having a liquid retention capacity of about 0.01 to about 25 microliters, more preferably about 1 to about 2 microliters.

The culture device can have, for example, about 10 to about 10,000 hydrophilic liquid-retaining zones, more preferably about 400 to about 600 hydrophilic liquid-retaining zones.

The hydrophilic liquid-retaining zones may comprise microvolume wells surrounded by a hydrophobic land area. Alternatively, the culture device may have a land area comprising a treated nanostructured film. In further alternative embodiments, the hydrophilic liquid-retaining zones may comprise hydrophilic fiber material projecting from the assay surface. The fiber material can be constructed of hydrophilic absorbent discs or of hydrophilic nonwoven fiber loop material.

In an alternative embodiment, the culture device may comprise a plurality of sets of hydrophilic liquid-retaining zones, each of the sets having zones of uniform size, the sets varying in liquid retention capacity, and the device having at least two sets of zones.

In another aspect, the invention features a culture device for detection or enumeration of microorganisms, the device comprising an assay surface, the assay surface comprising hydrophilic liquid-retaining zones and a hydrophobic land area between the zones, each zone having a microvolume capacity of liquid retention, and at least some of the zones comprising an assay reagent.

As used herein, the term "microorganism" includes all microscopic living organisms and cells, including without limitation bacteria, mycoplasmas, rickettsias, spirochetes, yeasts, molds, protozoans, as well as microscopic forms of eukaryotic cells, for example single cells (cultured or derived directly from a tissue or organ) or small clumps of cells. Microorganisms are detected and/or enumerated not only when whole cells are detected directly, but also when such cells are detected indirectly, such as through detection or quantitation of cell fragments, cell-derived biological molecules, or cell by-products.

The terms "hydrophobic" and "hydrophilic" are herein given the meanings commonly understood in the art. Thus, a "hydrophobic" material has relatively little or no affinity for water or aqueous media, while a "hydrophilic" material has relatively strong affinity for water or aqueous media. The relative hydrophobicities and hydrophilicities of the devices described herein are such as to ensure partitioning of liquid samples substantially into the described hydrophilic liquid-retaining zones upon application of the sample. The required levels of hydrophobicity and hydrophilicity may vary depending on the nature of the sample, but may be readily adjusted based on simple empirical observations of the liquid sample when applied to the devices.

Other advantages of the invention will be apparent from the following detailed description and the figures.
Figure 1 is a perspective view of one embodiment of an assay device.
Figure 2 depicts a top view of an assay device having sets of hydrophilic liquid-retaining zones varying in microvolume capacity of liquid retention.
Figure 3 is a schematic representation of an assay device including a hydrophobic nanostructured film.
Figure 4 is a schematic representation of an assay device in which the hydrophilic liquid-retaining zones are constructed of paper discs.
Figure 5a is a perspective view of an assay device in which the hydrophilic liquid-retaining zones are constructed of nonwoven fiber loop material.
Figure 5b is an expanded top view of the device depicted in Figure 5a.
Figure 6a is a photograph of a top view of an assay device in which the assay surface is hydrophilic.
Figure 6b is a photograph of a top view of an assay device with hydrophilic liquid-retaining zones and hydrophobic land areas.

This invention relates to partitioning of biological samples into microvolume liquid sample aliquots-for signal-based detection and enumeration of microorganisms in liquid samples.

Among the problems encountered in the art relating to the testing of liquid samples for microorganisms are relatively lengthy incubation times, the need to undertake multiple pipetting operations for aliquots being tested, and the need for a relatively large volume of sample for testing.

The present invention provides a solution to these and other problems associated with such testing. Methods and devices are provided for partitioning a liquid sample into microvolume compartments of a test device, with only minimal manipulation of the liquid sample required of the laboratory technician or other operator. A "microvolume," as that term is used herein, refers to a volume of less than about 25 microliters, and includes volumes in the sub-microliter range. The present inventors have discovered that the use of microvolumes in signal-based detection of microorganisms in liquid samples results in remarkably shorter incubation times required to produce a detectable signal. Because shorter incubation times are highly desirable in this field, this feature of the invention provides a distinct advantage.

In addition to achieving shorter incubation times, the use of microvolumes in the testing of liquid samples may allow for the use of substantially smaller test samples. Very small volume test samples are sometimes necessary due to very small volume sample sources. Small volume liquid test samples are also sometimes desirable, for example to ease handling or transport of the sample to a testing facility.

The present inventors have developed a number of novel devices for partitioning of biological liquid samples into discrete microvolumes within hydrophilic liquid-retaining zones (also referred to herein as "liquid-retaining zones" or "zones"). Non-limiting examples of these devices include: micro-embossed or pressed films having a plurality of microcompartments, for example microvolume wells, functioning as liquid-retaining zones, with the area between the wells ("land area") being hydrophobic and the wells being hydrophilic; nanostructured hydrophobic films in which discrete liquid-retaining zones of the film are hydrophilic and are adapted to retain microvolumes of a liquid sample for testing; and devices having hydrophilic liquid-retaining zones and hydrophobic land areas, where a given hydrophilic zone is fabricated from hydrophilic fiber material and projects upward or downward from the plane of the surrounding land area.

Advantageously, the above-summarized devices allow for the testing of liquid samples using microvolume aliquots in a single device, eliminating the need for separate vessels in such testing. A test sample may be distributed among hundreds or even thousands of discrete liquid-retaining zones, substantially increasing the number of data points in a test of the liquid sample.

A particularly useful application of these methods and devices is in the growth-based detection and enumeration of microorganisms in liquid test samples. Such growth-based detection and enumeration is very important in the testing of food, environmental, clinical, pharmaceutical, cosmetic, and other samples for contamination by microorganisms. The methods and devices of this invention allow for the efficient, accurate, convenient, and cost-effective testing of such samples.

A preferred use of the methods and devices of this invention in such microbiological testing is in MPN. In traditional MPN, a sample of interest is serially diluted (10 fold) and pipetted in equal amounts into replicate sets of tubes containing selective growth media and chemical indicators. The tubes are incubated at elevated temperature for about 24-48 hours followed by examination for growth of organisms. A statistical formula, based on the number of positive and negative tubes for each set, is used to estimate the number of organisms present in the initial sample. As currently used, this traditional method has several disadvantages. It is labor intensive because of the multiple diluting and pipetting steps required to perform the analysis. As a practical matter, only replicate sets of about three to five tubes for each dilution are commonly used. As a result, the 95% confidence limits for an MPN estimate of microbial concentration using this method are extremely wide. For example, a nine tube (3 ten-fold dilutions) MPN estimate of 20 has 95% confidence limits ranging from 7 to 89.

The use of the methods and devices of the present invention in MPN analysis overcomes several of the above-noted disadvantages. The amount of labor is greatly reduced because no pipetting into individual tubes is necessary, and very little or no agitation or other manipulations are required. Instead, the liquid sample is distributed to microvolume liquid-retaining zones by simply contacting the liquid sample with the device. In addition, fewer sample dilutions are necessary when large numbers of liquid-retaining zones are present in the device. The relatively large number of liquid-retaining zones also provides a more accurate estimate of microbial concentration. This is because the correspondingly larger number of data points provides a correspondingly narrower confidence limit interval.

Accordingly, the present invention provides a method for detecting (including enumerating) a microorganism in a liquid test sample. The method involves distributing microvolumes of the test sample to a plurality of hydrophilic liquid-retaining zones of an assay device. The assay device may be any device that includes an assay surface having a plurality of hydrophilic liquid-retaining zones, where each zone has a microvolume capacity of liquid retention. The device also includes a land area between the zones that is hydrophobic and remains substantially free of liquid after the biological sample has become distributed into the liquid-retaining zones. Non-limiting examples of such assay devices include those described herein.

The liquid-retaining zones in the assay device preferably are of uniform size and each zone has a liquid-retention capacity of about 0.01 to about 25 microliters of the liquid sample. Preferably, each zone has a liquid retention capacity of about 0.1 to about 10 microliters, and more preferably about 1 to about 2 microliters. The assay device preferably contains between 1 and about 100,000 liquid-retaining zones, more preferably about 10 to about 10,000 zones, even more preferably about 200 to about 5,000 zones and most preferably about 400 to about 600 zones.

The use of a device having about 400 to about 600 hydrophilic liquid-retaining zones is particularly useful in the context of testing a liquid sample for microorganism concentration using MPN. Certain regulatory requirements may dictate that a testing method must be able to detect one microorganism in a one-to-five-milliliter sample. Such a sample size is standard in the food processing industry for microbiological testing. Thus, for example, an assay device having 500 hydrophilic liquid-retaining zones, where each zone has a liquid capacity of about 2 microliters, would be very useful for testing a 1-ml sample. A liquid-retention zone having a capacity of 2 microliters allows for rapid development of a detectable signal in accordance with the invention, and the use of about 400 to about 600 zones provides a sufficiently large number of data points to substantially improve the confidence interval for an MPN calculation. In addition, it is feasible to perform a manual count of liquid-retaining zones testing positive for the microorganism. Use of devices having substantially more than 400 liquid-retaining zones may require, as a practical matter, instrument-assisted or automated counting.

The liquid test sample may be any sample of interest, from any source. The sample may be distributed to the plurality of liquid-retaining zones directly, or the sample may be diluted before distribution to the zones. The determination as to whether sample dilution is necessary will depend on a variety of factors such as sample source and age, and such determination is a routine matter to those of skill in the art.

The liquid test sample may include selective nutrient growth media for the microorganism of interest, and/or an indicator substance that produces a signal in the presence of the growing microorganism. Optionally, the nutrient medium may include a gelling agent that assists in "encapsulating" the growing microorganisms. Such gelling agents are known to those of skill in the art, and include any water-absorbing material that becomes a gel upon addition of an aqueous liquid.

Alternatively, one or both of the selective nutrient growth media and the indicator substance may be present as a coating or other deposition within a liquid-retaining zone, in amounts sufficient to achieve desired concentrations when a microvolume of the liquid test sample is distributed into the zone. Such a coating may be achieved, for example, by placing or distributing a solution of the nutrient medium (with or without gelling agent) and/or indicator substance into the liquid-retaining zone and drying the solution to produce a coating or deposition of the nutrient medium and/or indicator substance in the zone.

A wide variety of selective growth media for a wide variety of microorganisms of interest is known, as is a wide variety of indicator substances for a wide variety of microorganisms, and any of these media or indicator substances is suitable for use in the method of the invention. An advantage of the present invention is that soluble indicators can be used; since diffusion is prevented by confinement of the aqueous biological sample liquid in the hydrophilic liquid-retaining zones.

Various methods may be employed to distribute a liquid test sample to the liquid-retaining zones. More than one method may be applicable to a particular device, although the preferred method may depend to some extent on the configuration of a particular assay device. For example, for film devices containing hydrophilic microvolume wells or for devices in which the zones comprise hydrophilic fiber material projecting from the plane of the assay surface, the sample may be poured or pipetted over the device and the sample spread to the liquid-retaining zones by tilting or rocking the device. Alternatively, the assay surface of the device can be immersed in the sample as described in Example 4. Upon removal of the assay surface from the liquid sample, liquid is retained in the hydrophilic liquid-retaining zones and is likewise substantially excluded from the hydrophobic land area.

After the sample is distributed to the hydrophilic liquid-retaining zones of the assay device, various assays may be carried out depending on desired uses. For microbial detection or enumeration, the assay device may be incubated for a time sufficient to permit at least one cell division cycle of the microorganism. For these purposes, the device is generally incubated at about 25°C to about 45°C, more preferably at about 30°C to about 37°C. The incubation time for bacterial detection will vary. The detection time for most bacteria will range from about 20 minutes to about 24 hours in order to produce detectable growth as demonstrated by the indicator substance in the incubated liquid test sample. This relatively short incubation time represents a distinct advantage over detection methods currently used, which typically require incubation times of about 24 hours or more.

Following incubation of the assay device, the presence or absence of the microorganism in the liquid-retaining zones (and thus in the liquid test sample) is detected. The mode of detection depends on the type of indicator substance used in the method. Any indicator substance that is capable of providing a detectable signal may be used. Such indicators include but are not limited to fluorescent, chromogenic, luminescent, and electrochemical indicators. The presence or absence of a microorganism in a zone can be visually detected, with the naked eye or microscopically, if a chromogenic or luminescent indicator is used. If a fluorescent indicator substance is used, equipment and methods for detecting a fluorescent signal may be employed for detection. There are numerous indicator substances and signal detection systems, including systems for detecting electrochemical changes, known in the art for detecting microorganisms, and any such substance or system may be used in accordance with the present invention.

The detection of microorganisms in the liquid sample may further involve the enumeration of a microorganism count in the liquid test sample. In a preferred embodiment, the enumeration is performed using MPN. Once the number of liquid-retaining zones containing the microorganism of interest is determined, an MPN calculation can be made using known MPN techniques. If desired, the number of microorganisms in an individual zone can then be determined using known techniques, for example, signal intensity compared to a known standard, or by plating the contents of the zone. Advantageously, the large number of liquid-retaining zones used in the method of the invention allows for narrower intervals for the 95% confidence limits in an MPN analysis of a liquid test sample.

Because of the large number of liquid-retaining zones that may be manufactured in a single device, it is possible to use a single device in the detection and enumeration of multiple microorganisms of interest, while retaining the advantages of the invention. For example, a single liquid test sample can be tested for the presence or concentration of *E. coli* and *S. aureus*. One portion of an assay device can contain hydrophilic liquid-retaining zones for the detection and enumeration of one of these microorganisms, while a second set of zones can be directed to detection and enumeration of another microorganism of interest. This is accomplished, for example, by including microorganism-specific nutrients and/or indicator substances in the respective sets of liquid-retaining zones. Alternatively, all liquid-retaining zones can contain assay reagents designed for the simultaneous detection of multiple microorganisms. For example, *E. coli* can be detected with a fluorescent indicator substance while, at the same time, other coliforms are detected with a chromogenic indicator substance.

In another embodiment, the distribution step can involve distributing aliquots of the liquid test sample to a plurality of hydrophilic liquid-retaining zones of an assay device, wherein the assay device includes a plurality of sets of zones. Each set has zones of uniform size, and the device has at least two sets of zones. For example, the assay device can include a plurality of lanes, with the hydrophilic liquid-retaining zones in a particular lane having the same liquid-retention capacities. This feature allows for the distribution of the liquid test sample into different test volume sizes within a single assay device. In MPN, this feature provides a significant advantage in that, for a highly concentrated sample, an appropriate volume size may be selected and MPN analysis performed using a single distribution step in a single device without the need for serial dilutions.

As stated above, the methods of this invention may be practiced using any assay device containing hydrophilic liquid-retaining zones and a hydrophobic land area, depending on the particular embodiment being practiced. The present inventors have developed several novel devices suitable for use in the methods of this invention. The following are non-limiting examples of such devices.

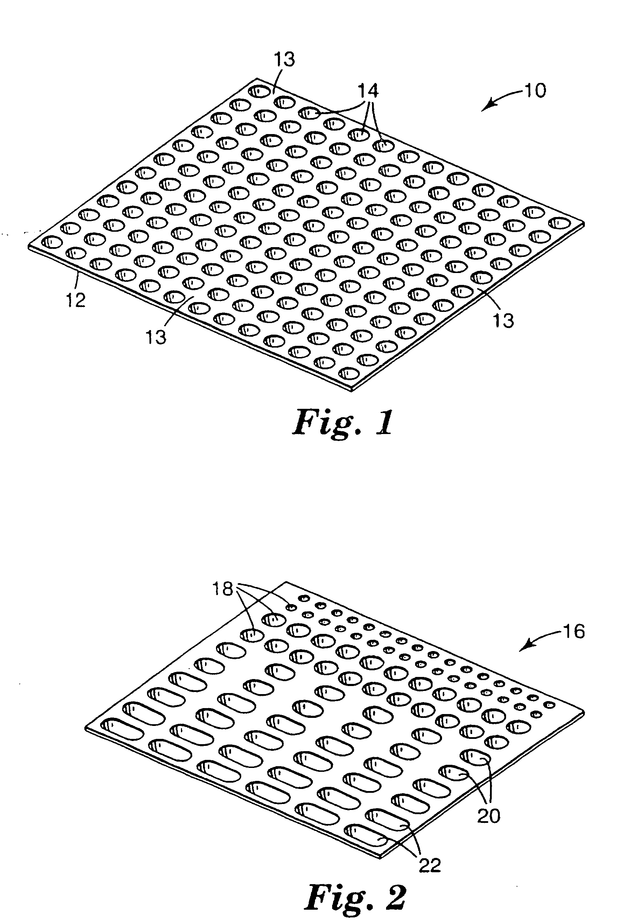

Referring to Figure 1, a device 10 comprises a substrate 12 having a plurality of hydrophilic liquid-retaining zones in the form of hydrophilic microvolume wells 14. The substrate 12 can be fabricated from any material in which microvolume wells can be fashioned and in which the microvolume wells retain their respective shapes throughout the useful life span of the device 10. Substrate 12 can be fabricated, for example, from polymeric films or other appropriate materials. Appropriate polymers include without limitation polyethylene, polypropylene, polyimides, fluoropolymers, polycarbonates, polyurethanes, and polystyrenes. Should a particular polymer not be sufficiently hydrophilic, it can be treated to impart hydrophilicity. For example, a surfactant can be included in the film to impart hydrophilicity. Those skilled in the art will recognize other means to impart surface hydrophilicity. Microvolume wells 14 can be formed by any process appropriate to the substrate 12 material. Such processes include without limitation thermal embossing, cast embossing, laser drilling, etching with reactive materials, or lamination of a sheet of patterned material containing a plurality of small openings onto a support film. Polyethylene or polypropylene films can be, for example, pressed embossed or extrusion embossed, and can include various pigments and surfactants.

Referring again to Figure 1, the area 13 between microvolume wells 14 ("land area") is fabricated to be hydrophobic. This serves to prevent aqueous liquid from bridging between the microvolume wells 14, thereby preventing cross-contamination. The land area 13 can be rendered hydrophobic in various ways. For example, the land area on an extrusion embossed polyethylene film, that had been rendered hydrophilic by incorporation of a surfactant, can be rendered hydrophobic by transferring a thin layer of acrylated silicone or other hydrophobic material to the land area.

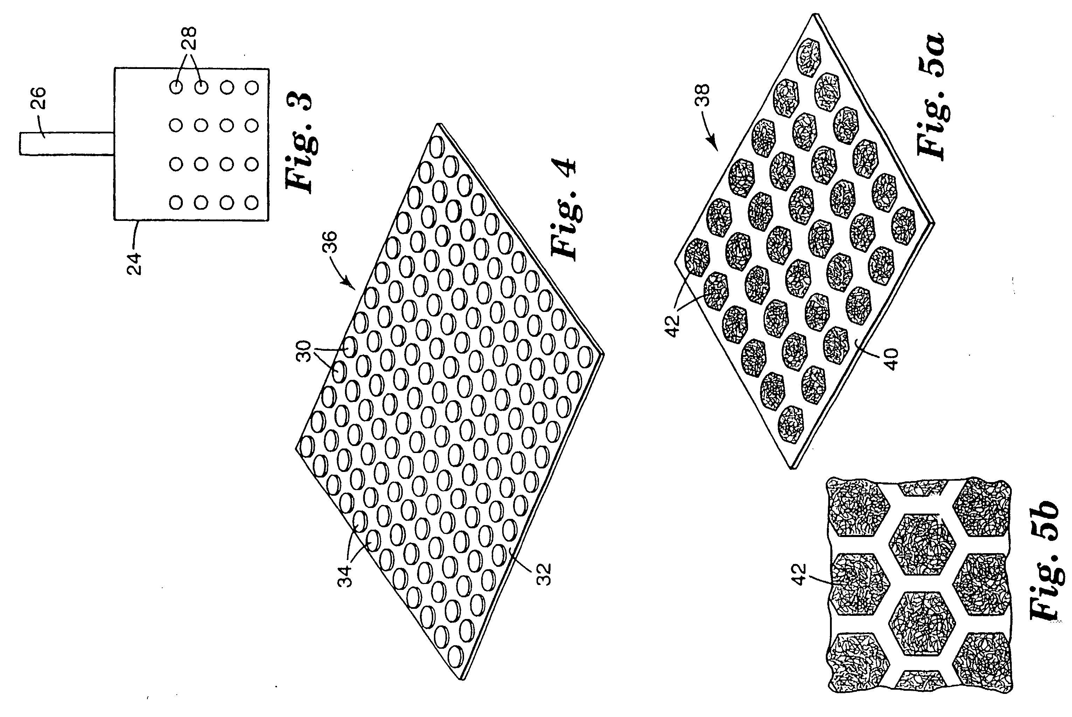

The device 10 can include any desired number of microvolume wells. Additionally, the device 10 can include relatively large reservoirs or other compartments adapted to hold larger volumes of liquid for maintenance of an appropriate humidity level within the device. Although the number of microvolume wells can be relatively small (e.g., 2-50) for certain applications such as preliminary screening, the small sizes of the microvolume wells allow relatively large numbers of wells to be fabricated on a single device 10. Preferably, the device has about 10 to about 10,000 liquid-retaining zones, even more preferably about 200 to about 5,000 zones, and most preferably about 400 to about 600 zones. The device 10 can have a population of uniformly sized microvolume wells 14, although the wells need not be of uniform size. For example, a device 16 as depicted in Figure 2 can have sets (e.g., rows) of microvolume wells in which volumes are constant within a set, but vary between sets. As depicted in Figure 2, the volumes can vary incrementally over an array of sets of wells, with the smaller wells 18 holding sub-microliter volumes and the larger wells 20 holding microliter volumes. It is even possible for the largest wells in a device such as depicted in Figure 2 to include wells 22 that would not be classified as "microvolume" wells. Such wells might have a liquid-retention capacity, for example, of substantially more than 25 microliters.

In an alternative embodiment, the substrate 12 can be coated with a hydrophobic nanostructured film. For example, polyimide or fluoropolymer webs can be vapor coated with organic pigments, lead, gold and other materials to create specific nanostructured films, then made hydrophobic by coating with an organized molecular assembly, such as octadecyl mercaptan or a fluorocarbon-hydrocarbon thiol, as described in Patent Application WO 96/34697. Relatively hydrophilic microvolume wells and other liquid-retaining zones may be fashioned by removing the hydrophobic nanostructured elements from selected areas of the substrate 12. This can be accomplished in various ways, including without limitation encapsulation/delamination and laser ablation as described in Example 3, below.

A representative hydrophobic nanostructured film device 24 is depicted schematically in Figure 3. Such devices can be loaded with sample simply by dipping in an aqueous sample solution. To this end, the device 24 can include a handle 26. Handle 26 allows an operator to place the device 24 in a liquid sample to any desired depth up to and including total immersion of the device 24 in the liquid sample, while avoiding contact of the operator's fingers with the sample. Upon removal of device 24 from the sample, liquid sample remains attached to the device only at the locations of the hydrophilic liquid-retaining zones 28. Incubation and detection are then performed as described above.

Assay devices also can be manufactured with hydrophilic liquid-retaining zones constructed of hydrophilic absorbent materials arrayed on a hydrophobic surface. For example, the zones can be constructed of absorbent papers having circular, oval, square, polygonal or other appropriate shapes. As illustrated in Figure 4, for example, discs of cotton linter binderless paper 30 can be laminated to a silicone-coated film 32 to form hydrophilic liquid-retaining areas 34 that project from the plane of the surrounding hydrophobic surface 36. Alternatively, the hydrophilic liquid-retaining zones may be constructed of nonwoven fiber loop material that likewise protrudes (projects) from the plane of the surrounding hydrophobic land area. For example, as illustrated in Figures 5a and 5b, the assay device 38 may comprise a sheet of hydrophobic polypropylene film 40 containing arrays of protrusions 42 fabricated from surfactant-containing polypropylene nonwoven fiber loop material.

Assay reagents can be coated or otherwise deposited within the liquid-retaining zones of the assay devices. Such assay reagents can include without limitation nutrients for growth of microorganisms; gelling agents; indicator substances such as chromogenic indicators, fluorescent indicators, luminescent indicators, and electrochemical indicators. The assay reagents can be immobilized in the liquid-retaining zones by any of numerous methods for immobilizing assay reagents on solid substrates known to those of skill in the art. Such methods include for example drying down assay reagent-containing liquids in the zones, as well as other methods for noncovalently attaching biomolecules and other assay reagents to a solid substrate. Alternatively, various methods may be employed to covalently attach assay reagents to the substrate 12 material within the wells 14 by methods well known to those of skill in the art.

As discussed above, the presence of hydrophilic liquid-retaining zones with microvolume liquid-retention capacity in an assay device allows for separation of a liquid test sample into a relatively large number of test volumes. The ability to separate a liquid sample into microvolume aliquots and to perform MPN or other assays without cross-contamination between aliquots is an advantage of the present method and devices.

Particular embodiments of this invention will be discussed in detail and reference has been made to possible variations within the scope of this invention. There are a variety of alternative techniques and procedures available to those of skill in the art which would similarly permit one to successfully practice the intended invention.

### EXAMPLES

The following examples are offered to aid in understanding of the present invention and are not to be construed as limiting the scope thereof. Unless otherwise indicated, all parts and percentages are by weight.

### Example 1

### Embossed Film Culture Devices

Embossed film culture devices containing a plurality of microcompartments and capable of being used for the detection of microorganisms in a liquid test sample were constructed as described in this example.

The hydrophilic liquid-retaining zones can be formed in a substrate by a number of processes, examples of which are thermal embossing, cast embossing, laser drilling, and by etching the surface with a reactive material. Detailed descriptions of how to make recesses or microvolume wells in polymeric films are provided in US-A-5,192,548; US-A-5,219,462; US-A-5,344,681; and US-A-5,437,754. The following descriptions are representative of specific embossed film culture devices used in the subsequent examples.

### A. Pressed Embossed Films Containing a Plurality of Microvolume Wells

Polyethylene (Eastman Chemical Company Resin #18BOA) containing 10% by weight TiO₂ (50% TiO₂/50% Polyethylene Pigment Concentrate) and 0.5% by weight Triton X-35 Surfactant (Sigma Chemical Company) or polypropylene was extrusion cast into a film (4-mil thickness). The film was cut into sheets and stacked (~20 sheets) onto photolithographically etched magnesium alloy tooling as described in U.S. Patent 5,219,462, designed to form a plurality of microvolume wells. The etched magnesium tooling contained protuberances arranged in the patterns described in subsequent examples. The stacked polyethylene sheets were embossed on a heated hydraulic press (232 °C, 1.4 N/m², 120 second dwell) as described in US-A-5,219,462. The samples were allowed to cool, at which time the tooling was removed to provide a single layer film containing the "negative" image of the tooling.

### B. Extrusion Embossed Films Containing a Plurality of Microvolume Wells

Photolithographically etched magnesium master tooling was attached to a steel roll using pressure-sensitive transfer adhesive. The polyethylene, pigment, and surfactant composition described in Example 1A was blended together and extrusion cast onto the roll as described in US-A-5,192,548. Embossed films lacking the Triton X-35 surfactant were also prepared in this manner.

### C. Extrusion Embossed Films with Hydrophobic "Land" Area

Extrusion embossed polyethylene films containing Triton X-35 Surfactant were prepared according to Example 1B. The area between microvolume wells ("land" area) was rendered hydrophobic by transferring a thin layer of acrylated silicone (Goldschmidt FC 711) containing 4.8% of a cross linking agent (Darocur 1173) with a roll-to-roll coating apparatus (Straub Design Co.). The hydrophobic coating was cured by exposing the film to ultraviolet radiation under nitrogen atmosphere using a Fusion Systems UV lamp with an H bulb providing a dosage of 85 millijoules/cm².

### Example 2

### Method of Inoculation

### (Method Utilizing Plurality of Microvolume Wells)

### A. Inoculation with Indicator Solution

An aqueous solution containing phenol red indicator (to provide contrast) was applied by pipette onto silicone-treated and silicone-untreated polyethylene embossed films (Examples 1C and 1B, respectively) containing a plurality of microvolume wells (about 1.3 µl/well). The microvolume wells were arranged in a hexagonal array (∼19 wells/cm²) and each well was in the shape of an inverted truncated cone, having a diameter of approximately 1.9 mm at the surface and 1.0 mm at its depth, which was about 1.1 mm. The microvolume wells were filled as described in US-A-5,219,462 by drawing the diluted sample solution down the film with the edge of a razor blade. The samples treated with the hydrophobic silicone coating were shown to partition liquid into individual microvolume wells without fluid bridging between the wells, whereas bridging of liquid was observed on the untreated films.

### B. Inoculation with Microorganism-Containing Samples

The method of inoculating embossed film culture devices containing a plurality of microvolume wells with bacteria-containing media was demonstrated in this example. The inoculated devices were utilized to detect and enumerate *E. coli* bacteria.

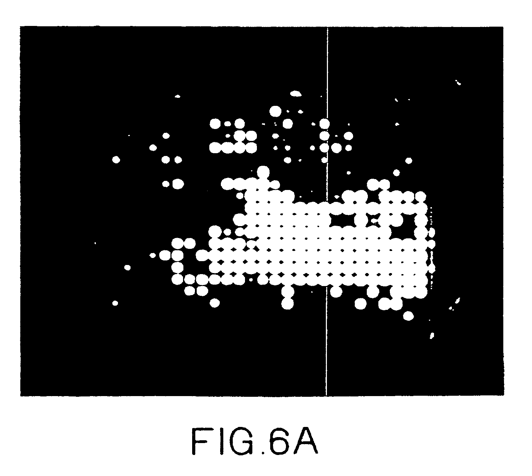

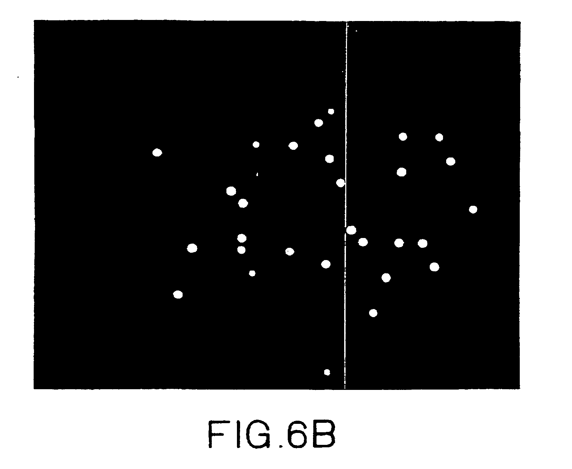

An overnight broth culture of *E. coli* ATCC 51813 (~10⁹ CFU/ml in Tryptic Soy Broth (TSB) media) was serially diluted into Violet Red Bile (VRB) media (7.0 g/l Bacto peptone, 3.0 g/l yeast extract, and 1.5 g/l bile salts) containing 4-methylumbelliferyl-β-D-glucuronide (0.5 mg/ml) (MUG, Biosynth International, Naperville, IL). The dilution was prepared to the approximate bacterial concentration of 100 CFU/ml. The diluted sample (0.5 ml) was applied by pipette onto silicone-treated and silicone-untreated polyethylene embossed films (406 microvolume wells) as described in Example 2A. The inoculated embossed films 43 were placed inside petri dishes, and incubated for 12 hours at 37°C. Twenty-eight microvolume wells 44 exhibited sharp, discrete fluorescent spots on the silicone-treated film 46 (Figure 6b). In contrast, significant well-to-well cross-contamination was observed on the untreated film (Figure 6a). For the silicone-treated film, 28 positive wells corresponds to a most probable number (MPN) of 58 CFU/ml, as calculated using the formula MPN= N ln (N/N-X) where N is the total number of filled wells and X is the total number of wells showing a positive reaction.

The results of this example show that microorganisms can be readily detected and enumerated using an embossed film culture device having a plurality of microvolume wells and that well-to-well cross-contamination can be eliminated by coating a hydrophobic substance on the land area between wells.

### Example 3

### Nanostructured Film Culture Devices

Nanostructured film culture devices containing a plurality of hydrophilic microvolume liquid-retaining zones arrayed on a substrate coated with a hydrophobic nanostructured film were constructed as described in this example.

### A. Nanostructured Film

Processes for generating nanostructured surfaces are disclosed in US-A-4,812,352 and US-A-5,039,561. Briefly, the organic pigment C.I. Pigment Red 149 (American Hoechst-Celanese, Somerset, NJ) was vacuum vapor deposited to a thickness of 250 nm onto a 0.0125-nm thick, 30 x 30 cm sheet of polyimide web, which had previously been metal vapor coated with 700 Å of platinum. The sample was annealed in a vacuum oven at 264 °C for greater than 30 minutes, which was sufficient to convert the PR 149 pigment to a dense distribution of discrete crystalline whiskers oriented perpendicular to the web substrate. The whiskers were vapor coated with a mass equivalent thickness of 2500 Å of gold, which resulted in a conformational coating of gold particles, ∼2 µm tall and ~0.15 µm in diameter, with an areal number density of 5 per (µm)², as determined by SEM.

Alternatively, the polyimide was replaced with a transparent fluorenone polyester (FPE, 3M Co.) and vapor coated with 50 Å of gold, which prevented surface charging during deposition of the PR 149, yet remained essentially transparent.

### B. Hydrophobic Nanostructured Film

The nanostructured film was then made hydrophobic by immersing in a 0.1mM solution of C₈F₁₇(CH₂)₁₁SH in ethanol for 4 hours, followed by rinsing with pure ethanol and air drying. The resulting highly hydrophobic surface was measured to have identical advancing and receding contact angles of 178 ° for water. This process is described in Patent Application WO 96/34697.

### C. Nanostructured Film Culture Devices

Nanostructured film culture devices were constructed by using an encapsulation/delamination of nanostructured films process described in US-A-5,336,558. Briefly, pieces of the nanostructured hydrophobic film were cut into 1.5 x 2.0 cm strips. A 0.25-mm thick perforated steel sheet, having a square array of 1.5-mm diameter holes spaced -4 mm apart, was laid over the nanostructured side of the strips. A fast curing vinyl polysiloxane encapsulate (3M EXPRESS dental impression material, 3M Co.) was applied liberally over the steel plate to cause the material to penetrate through the holes and encapsulate the nanostructured whiskers. After several minutes, the impression material was set and the steel sheet was removed, thereby removing the nanostructured elements cleanly from the polyimide web only at the location of the array of holes. The exposed metal-coated polyimide substrate in the areas under the holes was relatively hydrophilic compared to the remainder of the surface. This was demonstrated by dipping the strips into an aqueous solution and observing that small droplets remained attached only at the array of exposed spot or zone areas.

Alternatively and preferably, laser ablation was utilized for removing the nanostructured elements from the polyimide web to provide the desired array of relatively hydrophilic liquid-retaining zones. The strips of nanostructured hydrophobic film were ablated with a Nd-YAG laser with a collimated beam 1 mm in diameter and operated in a Q-switched mode with approximately 2 mJoule, 60 nanosecond pulses. Single pulses were used to ablate rows of 1-mm diameter zones on 4- and 5-mm center-to-center spacing. Larger zones, ∼1.6 x 1.6 mm square, were produced by overlapping a 3 x 3 matrix of nine 1-mm diameter zones. The resulting nanostructured film culture device with 40 (4 x 10) zones was submersed in water for 1 minute initially to make the ablated zone areas hydrophilic. Upon withdrawing the plate, each of the 40 zones had an -1-mm diameter, hemispherical droplet attached to it.

### Example 4

### Method of Inoculation

### (Method Utilizing Nanostructured Film Culture Devices)

### A. Inoculation with Aqueous Liquid Sample

To inoculate and measure the amount of liquid selectively retrieved by the nanostructured film culture devices (Example 3C), a plate with 12 hydrophilic liquid-retaining zones, ranging in size from 1 to 2.5 mm in diameter (average 2 mm), was dipped into pure water and the amount of water extracted onto the zones was measured gravimetrically. The plate was first dipped at a slow withdrawal rate of ~3 seconds/cm. After withdrawal, the back of the plate was touched against tissue paper to remove any water droplets clinging to the back of the polyimide plate, and the plate was then placed on a mass balance (0.1 mg minimum sensitivity) and the mass recorded 15 seconds later. This was repeated 15 times. The mean and standard deviation of the mass of the 12 water zones was 3.7 ± 0.2 mg, giving an average zone volume of 0.310 µl ± 5%. The procedure was then repeated a fast withdrawal rate with the plate pulled from the water in a time estimated to be ~0.1 second. At this rate, the amount of liquid that remained on the hydrophilic zones was larger, because the liquid did not have time to "stretch" and dynamically equilibrate. The mean and standard deviation of the 15 trials was 6.0 ± 0.5 mg, giving an average zone volume of 0.500 µl ± 12%.

### B. Inoculation with S. Aureus-Containing Samples

The method of inoculating nanostructured film culture devices containing a plurality of microvolume liquid-retaining zones with bacteria-containing media was demonstrated in this example. The inoculated devices were utilized to detect and enumerate *S. aureus* (Example 4B) and *E. coli* (Example 4C) bacteria.

A mixture (5 µl) of molten (∼60 °C) bacteriological growth media BHI (Brain Heart Infusion, Becton Dickinson and Co.) and agar (1.2% weight/volume) was spotted onto the hydrophilic zones of the nanostructured film culture devices prepared as described in Example 3C. The agar "spots" were allowed to cool and solidify at room temperature. One plate was dipped briefly into a growing culture of *Staphylococcus aureus* (~10⁸ cells/ml) in BHI broth medium. Other plates were dipped similarly into 1:10 and 1:1000 dilutions of the *S. aureus* culture, representing 10⁷ and 10⁵ cells/ml, respectively. The plates were placed into plastic petri dishes containing water-saturated filter paper to maintain humidity, and incubated at 37°C for 4 hours. The plates were then dipped into a solution containing 900 µl of HEPES Buffer (Sigma Chemical Co., pH 8.0); 120 µl of fluorescent indicator solution (1.0 mg/ml Boc-Val-Pro-Arg-AMC HCl (NovaBiochem, San Diego, CA) in 72 mM triethanolamine, 144 mM NaCl, pH 8.4); and, 30 µl human prothrombin (Sigma Chemical Co., 50 mg/ml in 5mM Tris buffer, 50mM NaCl, pH 8.0). The plates were incubated for one additional hour under the same conditions described above and then examined under UV light (∼366 nm, Mineralite, UVP, Inc., San Gabriel, CA). The zones containing agar media, bacterial suspension, and indicator solution all showed visible, intense bluish fluorescence as compared to no visible fluorescence in the control samples, which were prepared without any added bacteria. No cross-contamination between zones was observed.

### C. Inoculation with E. coli-Containing Samples

Agar medium was prepared by combining the following ingredients: pancreatic digest of gelatin (10 g, Peptone G, Acumedia Manufacturers, Inc., Baltimore, MD); Bacto Bile Salts Number 3 (2.5 g, Difco Labs, Detroit, MI); Agar (6 g, Difco Labs); and deionized water (500 ml). The mixture was stirred and heated to 100 °C until the agar melted, autoclaved at 121 °C for 15 minutes to sterilize, and then cooled to room temperature to solidify. An IPTG stock solution was prepared from filter-sterilized (0.2 mm) isopropyl-β-D-galactoside (IPTG, CalBiochem Corp., La Jolla, CA) in deionized water (200 mg/ml) and stored at -20 °C until use. A MU-Gal stock solution was prepared from 4-methylumbelliferyl-β-D-galactoside (MU-Gal) in N,N-dimethylformamide (10 mg/ml) and stored at 4 °C until use. Immediately before use the agar medium was melted at 100 °C and 25 ml was transferred to a sterile 50-ml tube. The IPTG stock solution (12.5 ml) and the MU-Gal stock solution (150 ml) were then mixed into the cooled (~60 °C) agar suspension. The mixture was immediately transferred (4-µl aliquots) to the nanostructured film culture device zones as described in Example 4B. After cooling to room temperature, the plates were dipped into a mid-exponential growing culture of *E. coli* ATCC 51813 (~10⁸ cells/ml in LB medium 3) and incubated in individual humidified petri dishes at 37°C. After 4 hours of incubation, the plates were checked for fluorescence with a Mineralite UV lamp. The inoculated zones exhibited slightly more fluorescence than that observed in the uninoculated zones. The plates were then incubated for an additional 16 hours and rechecked. The inoculated zones showed significantly more blue fluorescence than the uninoculated zones. The plate prepared with clear-film substrate (Example 3A utilizing FPE) was particularly convenient to measure because it could be illuminated from one side and viewed or photographed from the other side. No cross-contamination between zones was observed.

### Example 5

### Absorbent Disc Culture Devices

Absorbent disc culture devices containing a plurality of hydrophilic absorbent discs arrayed on a hydrophobic surface and capable of being used for the detection and enumeration of microorganisms in a liquid test sample were constructed as described in this example. A sheet of absorbent material (Scheicher & Schuell Grade 903 Paper; absorbs about 4.5 g of water/100 cm²) was laminated to a Rexam silicone-coated film (Grade #15819 D 2MIL CL PET MM34P/000 having a clear 2-mil thick polyester film as a substrate, Rexame Release, Oak Brook, IL) with an acrylate pressure sensitive adhesive (PSA) containing the chromogenic indicator 2,3,5-triphenyl-2H-tetrazolium chloride (TTC) (Amresco, Solon, OH). The material was saturated with TSB growth nutrient containing 0.5% of the fluorescent indicators 4-methylumbelliferyl phosphate (100 µg/ml, Sigma, St. Louis, MO) and 4-methylumbellifery-α-D-glucoside (50 µg/ml, Sigma), wiped with a wire-wound rod, and dried at 110°C for 10 minutes. Circular discs approximately 0.635 cm in diameter were punched out of the laminate and the silicone-coated film backing removed. The discs with PSA were then adhered to another sheet of Rexam silicone-coated film so that the discs were patterned in equally spaced parallel rows. The film and discs assemblies were gamma irradiated to a level of 8.9 kGy, cut to size, and then taped into a petri dish such that each dish contained a piece of film with 20 discs. Based on gravimetric measurements, each disc in the resulting culture devices had a capacity to retain about 40 µl of liquid.

### Example 6

### Method of Inoculation

### (Method Utilizing Absorbent Disc Culture Devices)

The method of inoculating absorbent disc culture devices containing a plurality of microvolume liquid-retaining zones (absorbent discs) with bacteria-containing media was demonstrated in this example. The inoculated devices were utilized to detect and enumerate *E. coli* bacteria.

A culture of *E. coli* ATCC 51813 was diluted to produce suspensions containing about 10 CFU/ml and 1 CFU/ml. Samples (1 to 2 ml) of the suspensions were applied by pipette to the absorbent disc culture devices described in Example 5. Excess liquid sample was poured off, thereby leaving about 0.8 ml retained on the device (20 discs, about 40 µl of liquid per disc). The inoculated devices were incubated at 35°C for 23 hours and inspected under ultraviolet light. The number of discs exhibiting fluorescence was counted for each device and most probable number (MPN) values calculated using the formula described in Example 2B. The MPN per milliliter was calculated by dividing the value obtained by the total volume of the sample (0.8 ml). Results are provided in Table 6a and are compared with counts obtained from standard testing with Coliform Count PETRIFILM™ Plates (3M Co.). The fluorescent discs often showed the red TTC color, usually as discrete spots within the discs. No cross-contamination between absorbent discs was observed.

**Table 6a**

| Enumeration of Microorganisms (*E. coli*) | | | |
|---|---|---|---|
| Bacterial Suspension (- CFU/ml) | Positive Discs (Out of 20) | MPN (CFU/ml) | Coliform Count PETRIFILM™ |
| 10 | 17 | 47 | 22 |
| 10 | 19 | 74 | 24 |
| 1 | 2 | 2.6 | 5 |
| 1 | 3 | 4.1 | 4 |

The results of this example show that absorbent disc culture devices having a plurality of absorbent discs arrayed on a hydrophobic film can be easily inoculated with bacteria-containing liquid samples and that the inoculated devices can be utilized for the detection and enumeration of *E. coli,* with the values obtained being comparable with those obtained from commercial Coliform Count PETRIFILM™ Plates.

### Example 7

### Method of Inoculation

### (Method Utilizing Hydrophilic Fiber Culture Devices)

The method of constructing and inoculating hydrophilic fiber culture devices containing a plurality of microvolume liquid-retaining zones (nonwoven fiber loops) with indicator solution and with bacteria-containing media were demonstrated in this example. The inoculated devices were utilized to detect and enumerate *E. coli* bacteria.

### A. Device Construction

A sheet of hydrophobic polypropylene film containing an array of relatively hydrophilic surfactant-containing polypropylene nonwoven fiber loop protrusions was prepared as described in US-A-5,256,231. The sheet was cut to size and taped to the bottom of a petri dish to form a culture device. Each device contained film having about 200 fiber loop protrusions patterned hexagonally in equally spaced parallel rows. Each hemispherical protrusion was hexagonal at its base (side length about 3 mm, height about 2 mm) and had a capacity to retain about 15 µl of liquid.

### B. Inoculation with Indicator Solution

A sample (1 ml) of phosphate buffer ("Butterfield", Fisher Scientific) containing phenol red indicator (to provide contrast) was applied by pipette onto the film in the center of the device. The liquid was observed to wick into the hydrophilic fiber loop protrusions radially from the point of inoculation. The liquid was observed to quickly partition into the loop protrusions while "draining" from the hydrophobic polypropylene land areas. About 65 of the 200 protrusions were filled. No bridging of the colored liquid across the land areas between loop protrusions was observed.

### C. Inoculation with Microorganism-Containing Sample

An overnight culture of *E. coli* (ATCC 51813, ~10⁹ CFU/ml in TSB media) was serially diluted into VRB Media (7.0 g/l Bacto peptone, 3.0 g/l yeast extract, 1.5 g/l bile salts) containing 4-methylumbelliferone-β-D-glucuronide (0.5 mg/ml). A dilution of 10⁻⁸ was prepared corresponding to a bacterial concentration of about 10 CFU/ml. A sample (1 ml) was pipetted onto the film in the center of a hydrophilic fiber culture device (Example 7A) as described in Example 7B. After inoculation, the petri dish was covered and sealed using electrical tape to prevent evaporation. The device was then inverted and incubated at 37°C for 19 hours. After incubation, the number of protrusions exhibiting fluorescence under 365 nm irradiation were counted. Five separate, discrete protrusions were observed to have significant fluorescence. No fluorescence was observed between protrusions, thereby indicating no cross-contamination. The MPN value was calculated to be 5 CFU/ml, using the formula described in Example 2B.

The results of this example show that hydrophilic fiber culture devices having a plurality of hydrophilic fiber zones arrayed on a hydrophobic film can be easily inoculated with bacteria-containing liquid samples and that the inoculated devices can be utilized for the detection and enumeration of *E. coli.*

Various modifications and alterations of this invention will be apparent to those skilled in the art without departing from the scope of this invention, and it should be understood that this invention is not limited to the illustrative embodiments set forth herein.

## Claims

1. A method for conducting a most probable number analysis, wherein an aqueous liquid sample is partitioned into discrete volumes, the method comprising:
a) providing a device for culturing a microorganism, said device having an assay surface, said assay surface comprising hydrophilic liquid-retaining zones and a hydrophobic land area between said zones;
b) contacting said liquid sample with said assay surface such that said liquid sample partitions into said hydrophilic liquid-retaining zones;
c) incubating said device;
d) detecting a signal that indicates microorganisms are growing in a zone; and
e) conducting a most probable analysis based on the number of zones wherein a signal is detected.

2. The method of claim 1, wherein said zones comprise a coating or deposition of assay reagent.

3. The method of claim 2, wherein said assay reagent comprises a nutrient medium.

4. The method of claim 2 or 3, wherein said assay reagent comprises at least one indicator substance and said indicator substance is selected from the group consisting of a chromogenic indicator, a fluorescent indicator, a luminescent indicator and an electrochemical indicator.

5. The method of any one of the preceding claims, wherein said culture device comprises about 400 to about 600 hydrophilic liquid-retaining zones.

6. A culture device suitable for practicing the method of any one of the preceding claims, said device comprising an assay surface, said assay surface comprising hydrophilic liquid-retaining zones (14; 18, 20, 22; 28; 34) and a hydrophobic land area (13) between said zones, at least some of said zones comprising an assay reagent wherein said assay reagent comprises a nutrient medium and said assay reagent is present as a coating in said hydrophilic liquid-retaining zones.

7. The culture device of claim 6, wherein said device has about 10 to about 10,000 zones (14; 18, 20, 22; 28; 34).

8. The culture device of claim 5 or 7, wherein each said zone (14; 18, 20, 22; 28; 34) has a liquid retention capacity of about 0.01 to about 25 microliters.

9. The culture device of any one of claims 5 to 8, wherein said assay reagent comprises an indicator substance.

10. The culture device of any one of claims 6 to 9, wherein said assay reagent comprises a nutrient medium.

11. The culture device of claim 9 or 10, wherein said indicator substance is selected from the group consisting of a chromogenic indicator, a fluorescent indicator, a luminescent indicator and an electrochemical indicator.

12. The device of any one of claims 6 to 11, wherein said liquid-retaining zones (34) comprise absorbent discs (30).

## Patentansprüche

1. Verfahren zur Durchführung von Analysen zur Bestimmung der wahrscheinlichsten Anzahl (most probable number), wobei eine wässrige flüssige Probe in einzelne Volumen aufgeteilt wird, aufweisend:
a) Bereitstellen einer Vorrichtung zum Kultivieren der Mikroorganismen, wobei die Vorrichtung eine Auswertungsoberfläche hat, wobei die Auswertungsoberfläche hydrophile flüssigkeitszurückhaltende Zonen aufweist, und eine hydrophobe erhöhte Fläche zwischen den Zonen;
b) in Kontakt bringen der flüssigen Probe mit der Auswertungsoberfläche derart, dass die flüssige Probe sich in die hydrophilen flüssigkeitszurückhaltenden Zonen verteilt;
c) Inkubieren der Vorrichtung;
d) Erfassen eines Signals, das zeigt, dass in einer Zone Mikroorganismen wachsen; und
e) Durchführen einer Analyse der wahrscheinlichsten Anzahl (most probable number) basierend auf der Anzahl der Zonen, in denen ein Signal erfasst wird.

2. Verfahren nach Anspruch 1, wobei die Zonen eine Beschichtung oder ein Depot von Auswertungsreagens aufweisen.

3. Verfahren nach Anspruch 2, wobei das Auswertungsreagens ein Nährmedium aufweist.

4. Verfahren nach Anspruch 2 oder 3, wobei das Auswertungsreagens mindestens eine Indikatorsubstanz aufweist, und die Indikatorsubstanz gewählt ist aus der Gruppe, die gebildet wird von einem Farbindikator, einem fluoreszierenden Indikator, einem lumineszierenden Indikator und einem elektrochemischen Indikator.

5. Verfahren nach irgendeinem der vorhergehenden Ansprüche, wobei die Kultiviervorrichtung etwa 400 bis etwa 600 hydrophile flüssigkeitszurückhaltende Zonen aufweist.

6. Kultiviervorrichtung, die zur Durchführung des Verfahrens eines der vorhergehenden Ansprüche geeignet ist, wobei die Vorrichtung eine Auswertungsoberfläche aufweist, wobei die Auswertungsoberfläche hydrophile flüssigkeitszurückhaltende Zonen (14; 18, 20, 22; 28; 34) aufweist, und eine hydrophobe erhöhte Fläche (13) zwischen den Zonen, wobei mindestens einige der Zonen ein Auswertungsreagens aufweisen, wobei das Auswertungsreagens ein Nährmedium aufweist, und das Auswertungsreagens als Beschichtung in den hydrophilen flüssigkeitszurückhaltenden Zonen vorliegt.

7. Kultiviervorrichtung nach Anspruch 6, wobei die Vorrichtung von etwa 10 bis etwa 10 000 Zonen (14; 18, 20, 22; 28; 34) hat.

8. Kultiviervorrichtung nach Anspruch 5 oder 7, wobei jede Zone (14; 18, 20, 22; 28;34) eine Flüssigkeitsrückhaltekapazität von etwa 0,01 bis etwa 25 Mikroliter hat.

9. Kultiviervorrichtung nach einem der Ansprüche 5 bis 8, wobei das Auswertungsreagens eine Indikatorsubstanz aufweist.

10. Kultiviervorrichtung nach einem der Ansprüche 6 bis 9, wobei das Auswertungsreagens ein Nährmedium aufweist.

11. Kultiviervorrichtung nach Anspruch 9 oder 10, wobei die Indikatorsubstanz gewählt ist aus der Gruppe, die gebildet wird von einem Farbindikator, einem fluoreszierenden Indikator, einem lumineszierenden Indikator und einem elektrochemischen Indikator.

12. Vorrichtung nach einem der Ansprüche 6 bis 11, wobei die flüssigkeitszurückhaltenden Zonen (34) absorbierende Scheiben (30) aufweisen.

## Revendications

1. Procédé de conduite d'une analyse du nombre le plus probable, dans lequel un échantillon liquide aqueux est divisé en volumes discrets, le procédé consistant à :
a) fournir un dispositif pour la mise en culture d'un micro-organisme, ledit dispositif comportant une surface d'analyse, ladite surface d'analyser comprenant des zones hydrophiles de retenue des liquides et une zone plate hydrophobe entre lesdites zones ;
b) mettre en contact ledit échantillon liquide avec ladite surface d'analyse, de sorte que ledit échantillon liquide soit divisé et introduit dans lesdites zones hydrophiles de retenue des liquides ;
c) mettre en incubation ledit dispositif ;
d) détecter un signal qui indique que des micro-organismes sont en train de se multiplier dans une zone ; et
e) conduire une analyse du nombre le plus probable basée sur le nombre de zones dans lesquelles un signal est détecté.

2. Procédé selon la revendication 1, dans lequel lesdites zones comprennent un revêtement ou un dépôt de réactif d'analyse.

3. Procédé selon la revendication 2, dans lequel ledit réactif d'analyse comprend un milieu nutritif.

4. Procédé selon la revendication 2 ou 3, dans lequel ledit réactif d'analyse comprend au moins une substance indicatrice et ladite substance indicatrice est sélectionnée dans le groupe constitué d'un indicateur chromogène, d'un indicateur fluorescent, d'un indicateur luminescent et d'un indicateur électrochimique.

5. Procédé selon l'une quelconque des revendications précédentes, dans lequel ledit dispositif de mise en culture comprend entre environ 400 et environ 600 zones hydrophiles de retenue des liquides.

6. Dispositif de mise en culture convenant à une mise en oeuvre du procédé selon l'une quelconque des revendications précédentes, ledit dispositif comprenant une surface d'analyse, ladite surface d'analyse comprenant des zones hydrophiles de retenue des liquides (14 ; 18, 20, 22 ; 28 ; 34) et une zone plate hydrophobe (13) entre lesdites zones, quelques-unes au moins desdites zones comprenant un réactif d'analyse dans lequel ledit réactif d'analyse comprend un milieu nutritif et ledit réactif d'analyse est présent sous la forme d'un revêtement dans lesdites zones hydrophiles de retenue des liquides.

7. Dispositif de mise en culture selon la revendication 6, dans lequel ledit dispositif comporte entre environ 10 et environ 10 000 zones (14 ; 18, 20, 22 ; 28 ; 34).

8. Dispositif de mise en culture selon la revendication 5 ou 7, dans lequel chacune desdites zones (14 ; 18, 20, 22 ; 28 ; 34) a une capacité de rétention de liquide entre environ 0,01 et environ 25 microlitres.

9. Dispositif de mise en culture selon l'une quelconque des revendications 5 à 8, dans lequel ledit réactif d'analyse comprend une substance indicatrice.

10. Dispositif de mise en culture selon l'une quelconque des revendications 6 à 9, dans lequel ledit réactif d'analyse comprend un milieu nutritif.

11. Dispositif de mise en culture selon la revendication 9 ou 10, dans lequel ladite substance indicatrice est sélectionnée dans le groupe constitué d'un indicateur chromogène, d'un indicateur fluorescent, d'un indicateur luminescent et d'un indicateur électrochimique.

12. Dispositif selon l'une quelconque des revendications 6 à 11, dans lequel lesdites zones de retenue des liquides (34) comprennent des disques absorbants (30).
